(19)

**Europäisches Patentamt**
**European Patent Office**
**Office européen des brevets**

(11)  **EP 3 342 416 A1**

(12)  **EUROPÄISCHE PATENTANMELDUNG**

(43) Veröffentlichungstag:
    **04.07.2018   Patentblatt 2018/27**

(51) Int Cl.:
    *A61K 36/484* (2006.01)          *A61P 39/00* (2006.01)
    *A61P 39/02* (2006.01)

(21) Anmeldenummer: **16450032.4**

(22) Anmeldetag: **28.12.2016**

(84) Benannte Vertragsstaaten:
    **AL AT BE BG CH CY CZ DE DK EE ES FI FR GB GR HR HU IE IS IT LI LT LU LV MC MK MT NL NO PL PT RO RS SE SI SK SM TR**
    Benannte Erstreckungsstaaten:
    **BA ME**
    Benannte Validierungsstaaten:
    **MA MD**

(71) Anmelder: **Erber Aktiengesellschaft**
    **3131 Getzersdorf bei Traismauer (AT)**

(72) Erfinder:
    • **MAYER, Elisabeth**
      **3481 Fels am Wagram (AT)**

    • **NOVAK, Barbara**
      **3430 Tulln an der Donau (AT)**
    • **SCHWAB-ANDICS, Christina**
      **1090 Wien (AT)**
    • **HOFSTETTER-SCHÄHS, Ursula**
      **1140 Wien (AT)**
    • **SCHATZMAYR, Gerd**
      **3430 Tulln (AT)**

(74) Vertreter: **Cunow, Gerda**
    **Cunow Patentanwalts KG**
    **Teschnergasse 33/1/3**
    **1180 Wien (AT)**

(54)  **VERWENDUNG VON WENIGSTENS EINEM GLYCYRRHIZA-PFLANZENPRÄPARATS, EINES ANTIDOS DAVON SOWIE VERWENDUNG DES ANTIDOTS**

(57)   Verwendung von wenigstens einem Glycyrrhiza-Pflanzenpräparat gewählt aus der Gruppe Mehl aus einer gesamten getrockneten Glycyrrhiza-Pflanze, Mehl aus Blättern der getrockneten Glycyrrhiza-Pflanze, Mehl aus Wurzeln der getrockneten Glycyrrhiza-Pflanze, wässriger Trockenextrakt aus der Glycyrrhiza-Pflanze, wässrig/ethanolischer Trockenextrakt aus der Glycyrrhiza-Pflanze, wässriger Extrakt aus der Glycyrrhiza-Pflanze, gegebenenfalls gemeinsam mit wenigstens einem Hilfsstoff zur Reduktion der toxischen Wirkung von wenigstens einem Polypeptid-Fungitoxin in agrarischen Produkten.

**EP 3 342 416 A1**

## Beschreibung

Die vorliegende Erfindung bezieht sich auf die Verwendung von wenigstens einem Glycyrrhiza-Pflanzenextrakt sowie ein Antidot zur oralen Einnahme zur Reduktion der toxischen Wirkung von wenigstens einem Polypeptid-Fungitoxin ebenso wie die Verwendung des Antidots.

Glycyrrhiza- bzw. Süßholz-Pflanzenpräparate werden schon seit der Antike als Heilmittel eingesetzt und werden auch in der traditionellen chinesischen Medizin als eine der 50 Basiskräuter erwähnt. Glycyrrhiza ist ein aus dem griechischen stammender Begriff, der bereits die Haupteigenschaft der Wurzel dieser Pflanze beschreibt, nämlich dass sie süß schmeckt und dass es sich überdies um die Wurzel handelt, hierbei bedeutet "glycos" süß und "rhiza" Wurzel. Unter allen Glycyrrhiza-Spezies ist wirtschaftlich *Glycyrrhiza glabra* wahrscheinlich am relevantesten und stellt neben *Glycyrrhiza uralensis*, welche vor allem in der traditionellen chinesischen Medizin eingesetzt wird, den wichtigsten Vertreter der Süßhölzer dar. Seit langer Zeit sind die in der Süßholzwurzel enthaltenen Stoffe unter anderem für ihre entzündungshemmenden und schleimlösenden Wirkungen bekannt, wobei von den wirksamkeitsbestimmenden Inhaltsstoffen der Süßholzwurzel vor allem die Glycyrrhizinsäure, insbesondere 18-beta-Glycyrrhizinsäure, eine Hauptkomponente ausmacht. Gehalte an Glycyrrhizinsäure schwanken hierbei je nachdem, wie die Süßholzwurzel behandelt wird, und woher die Pflanze kommt, stark. Sie können je nach Behandlung unterschiedliche Gehalte an Glycyrrhizinsäure aus derselben Wurzel gewonnen werden. Als weitere wichtige Inhaltsstoffe der Süßholzwurzel sind etwa 9% stickstoffhaltige Substanzen, bis zu 3,5% Fett, geringe Gehalte an Gerbstoffen, bis über 30% Stärke, ein ätherisches Öl, L-Asparagin, bis zu 10% Bitterstoffe und etwa 4% Harze, Apfelsäure und Oxalsäure identifiziert worden.

Ein wichtiger Bestandteil der Süßholzwurzel, die Glycyrrhizinsäure wurde unter anderem von Liu et al. (Zhongguo Zhong Yao Za Zhi, 2014, 39(19), 3841ff) in Bezug auf ihren Effekt auf Lipopolysaccharide (LPS) induzierte Cytokinexpression in Makrophagen untersucht und festgestellt, dass dieser eine antiinflammatorische Wirkung besitzt.

Fungitoxine sind giftige, sekundäre Stoffwechselprodukte die von Schimmelpilzen gebildet werden. Abhängig von ihrer Art und Konzentration im Futter wirken sie sich negativ auf die Leistung und die Gesundheit von Nutztieren aus, indem sie unter anderem Fungitoxin-Toxikosen auslösen können. Zu den negativen Effekten zählen unter anderem Leistungsverlust, Übelkeit, Durchfall, verringerte Fruchtbarkeit, Schwächung des Immunsystems, Entstehung von Krebs und die Schädigung des Nervensystems. Fungitoxine stellen somit ein gesundheitliches Risiko und in der Folge ein wenigstens ebenso großes wirtschaftliches Risiko dar.

Dies wird noch dadurch verstärkt, dass Schimmelpilze gleichzeitig verschiedene Fungitoxine und sekundäre Metaboliten bilden können. Daher werden mit den heutigen, sehr genauen Analysemethoden meist mehrere Fungitoxine und sekundäre Metabolite in Rohstoffen und damit auch in Futtermittelproben detektiert. Streit et al., Toxins, 2013, 5, 504 ff hat in über 90% der getesteten Rohstoffe und Futtermittelproben Fungitoxine und sekundäre Metabolite gefunden. 7 - 69 Metaboliten pro Probe wurden detektiert. Bereits sehr niedrige Konzentrationen von gleichzeitig vorkommenden Fungitoxinen können aufgrund des Vorhandenseins von nicht näher untersuchten synergistischen Effekten eine negative Wirkung auf Nutztiere haben.

Verschiedene Gattungen von Schimmelpilzen produzieren für die Landwirtschaft schädliche Fungitoxine, wie zum Beispiel *Aspergillus, Fusarium und Penicillium* (Frisvad et al, Adv. Exp. Med. Biol., 2006, 571, 3ff). Die bekanntesten und meistuntersuchten Schimmelpilzgifte im Bereich der Tierernährung sind Aflatoxine, z.B. Aflatoxin B1, Trichothecene, z.B. Deoxynivalenol, Zearalenon, Ochratoxin A und Fumonisine, z.B. Fumonisin B1.

Mittlerweile sind mehr als 500 verschiedene Fungitoxine und sekundäre Metabolite davon bekannt, wobei Beauvericin (CAS-Nr: 26048-05-5), Enniatine (CAS-Nr: 11113-62-5) wie Enniatin A (CAS-Nr: 2503-13-1), A1 (CAS-Nr: 4530-21-6), B (CAS-Nr: 917-13-5), B1 (CAS-Nr: 19914-20-6), B2, B3 und Apicidin (CAS-Nr: 183506-66-3) wichtige Vertretern sind. In der Arbeit von Streit et al. (Toxins, 2012, 4, 788ff) wurde Beauvericin in 89%, verschiedene Enniatine in 96% und Apicidin in 66% der Proben gefunden. Enniatine konnten in 37%, 68% bzw. 76% der getesteten Lebensmittel- (n = 4,251), Futtermittel- (n = 3,640) bzw. in 141 verschiedenen unverarbeiteten Getreide- (n = 2,647) proben nachgewiesen werden, Beauvericin wurde hingegen in 20%, 21% bzw. 54% der getesteten Lebensmittel- (n = 732), Futtermittel- (n = 861) bzw. 198 verschiedenen unverarbeiteten Getreide- (n = 554) proben detektiert. Alle Proben wurden in Europa im Zeitraum zwischen dem Jahr 2000 und 2013 gesammelt (EFSA Journal, 2014, 12, 3802). Enniatine (ENNs), Beauvericin (BEA) und Apicidin (API) können anhand ihrer Syntheseart in eine gemeinsame Gruppe eingeordnet werden. Sie entstehen durch die Peptidbiosynthese und haben eine Polypeptidstruktur gemeinsam. Enniatine und Beauvericin sind symmetrisch aufgebaut und weisen 3 Peptidbindungen auf, wobei sich Ester und Amidbindung abwechseln. Apicidin ist nicht symmetrisch aufgebaut, es weist 4 Peptidbindungen auf, bei welchen es sich um Amidbindungen handelt. Enniatine, Beauvericin und Apicidin werden im Folgenden als Untergruppe der Fungitoxine gemeinsam als "Polypeptid-Fungitoxine" bezeichnet. Die durch die Polypeptid-Fungitoxine in Subjekten wie Tiere und Menschen verursachten Störungen bzw. Erkrankungen werden hierin als "Polypeptid-Fungitoxin-Toxikosen" bezeichnet. Im Gegensatz dazu werden Deoxynivalenol und Aflatoxin B1 durch einen grundlegend unterschiedlichen Weg, nämlich durch Isobutyl-Biosynthese hergestellt und besitzen eine Poly-Isoprenstruktur.

Polypeptid-Fungitoxin-Toxikosen verursacht durch Enniatine, Beauvericin und Apicidin:

Von Enniatinen ist bekannt, dass sie in *in vitro* Versuchen zytotoxische Wirkungen auf Säugetierzelllinien zeigen. Hohe Enniatin-Gehalte konnten im Jejunum, der Leber und im Fettgewebe von Ratten detektiert werden, woraus geschlossen werden kann, dass die höchste Absorption im Jejunum bzw. Dünndarm stattfindet. McKee et al. (J. Nat. Prod, 1997, 60, 431ff) zeigten, dass hohe Dosen von Enniatinen für Versuchsmäuse in einer Studie tödlich waren, wobei selbst niedrigere Dosen zu einem Gewichtsverlust führten.

Beauvericin steht, zumindest für Geflügel, im Zusammenhang mit einer Erhöhung des Herzgewichtes. Bereits niedrige Konzentrationen von Beauvericin verursachen toxische Effekte in *in vitro* Versuchen.

Park et al. (Appl. Environ. Microbiol., 1999, 86, 126ff) zeigte, dass Apicidin, welches ein zyklischer Histon-Deacetylase-Inhibitor ist, bei Ratten zu toxischen Effekten führt, erkennbar durch Gewichtsverlust, Blutungen im Magen, Darm, und Blase und anschließendem Tod. Des Weiteren konnten antiproliferative und zytotoxische Effekte in Säugetierzelllinien nachgewiesen werden.

Allgemeine Symptome von Polypeptid-Fungitoxin-Toxikosen bei Nutztieren, insbesondere bei Schweinen und Geflügel, können Fressunlust und Durchfall sein, die sich negativ auf Leistungsparameter wie Lebendgewicht, Futterverwertungsrate oder Eigewicht auswirken.

Der erste Schritt zur Vermeidung der schädlichen Toxine und zwar sowohl von Fungitoxinen als auch Polypeptid-Fungitoxinen ist die Anwendung von geeigneten landwirtschaftlichen Praktiken sowie guten Lagerbedingungen agrarischer Produkte. Die Analysen von Futterproben zeigen jedoch, dass diese Maßnahmen nicht ausreichend sind. Zum Schutz der Tiere vor den negativen Auswirkungen der Fungitoxine auf Gesundheit und Leistungsfähigkeit werden Futtermitteladditive eingesetzt. Hier kommen unterschiedliche Inhaltsstoffe zur Anwendung.

Wirksame Futtermitteladditive können bereits bei Fungitoxin Kontaminierung von Futter mit Aflatoxinen, Zearalenonen, Trichothecenen, Ochratoxin A und Fumonisinen eingesetzt werden. Bisher sind jedoch keine Futtermitteladditive mit Wirksamkeit auf die Polypeptid-Fungitoxine Beauvericin, Enniatine und/oder Apicidin bekannt.

Es besteht somit ein wesentliches Bedürfnis, den Gehalt an Polypeptid-Fungitoxinen in Nahrungs- und Futtermitteln soweit als möglich zu reduzieren bzw. darin enthaltene Polypeptid-Fungitoxine durch Einsatz von Futtermitteladditiven bzw. Substanzen oder Substanzgruppen, welche derartige Polypeptid-Fungitoxine absorbieren oder abbauen bzw. unschädlich machen können, so weit als möglich zurückzudrängen.

Zur Lösung dieser Aufgabe wird gemäß der vorliegenden Erfindung wenigstens ein Glycyrrhiza-Pflanzenpräparat gewählt aus der Gruppe Mehl, wässriger Extrakt, wässriger/ ethanolischer Extrakt, wässriger Trockenextrakt und wässriger/ethanolischer Trockenextrakt aus einer gesamten Glycyrrhiza-Pflanze oder aus Wurzeln der Glycyrrhiza-Pflanze, gegebenenfalls gemeinsam mit wenigstens einem Hilfsstoff, zur Reduktion der toxischen Wirkung von wenigstens einem Polypeptid-Fungitoxin in agrarischen Produkten verwendet. Überraschender Weise hat es sich gezeigt, dass, wenn ein Glycyrrhiza-Pflanzenpräparat gewählt aus der Gruppe Mehl, wässriger Extrakt, wässriger/ethanolisher Extrakt, wässriger Trockenextrakt und wässriger/ethanolischer Trockenextrakt aus einer gesamten Glycyrrhiza-Pflanze oder aus Wurzeln der Glycyrrhiza-Pflanze eingesetzt wird, es gelingt, die toxische Wirkung von Polypeptid-Fungitoxinen in agrarischen Produkten so weit zurückzudrängen, dass eine Gefährdung für ein diese verzehrendes Subjekt, insbesondere Menschen oder Tiere nicht mehr besteht, insbesondere wesentlich reduziert wird.

Eine nahezu vollständige, insbesondere wesentliche Reduktion der toxischen Wirkung von wenigstens einem Polypeptid-Fungitoxin gewählt aus der Gruppe der Enniatine, Apicidin oder Beauvericin kann erreicht werden, wenn die Glycyrrhiza-Pflanze aus der Gruppe von Glycyrrhiza glabra und Glycyrrhiza uralensis verwendet wird.

Bei Verwendung eines wässrigen Trockenextrakts der Glycyrrhiza-Pflanze, insbesondere der Wurzeln der Glycyrrhiza glabra-Pflanze, welcher zwischen 4% (w/w) und 10% (w/w), insbesondere 7% (w/w) Glycyrrhizinsäure enthält, konnte, obwohl bekannt ist, dass die Glycyrrhizinsäure selbst keine Wirkung besitzt, und die eingesetzte Glycyrrhizinsäure Menge daher nur als Äquivalent zum Wirkbestandteil gesehen wird, überraschenderweise eine noch weitere Reduktion der Effekte von spezifischen Polypeptid-Fungitoxinen, nämlich von Beauvericin, Enniatinen und auch von Apicidin erreicht werden. Hierbei gelingt es durch Verwendung von wenigstens einem Glycyrrhiza-Pflanzenpräparat gemäß der vorliegenden Erfindung die toxische Wirkung von wenigstens einem spezifischen Polypeptid-Fungitoxin, gewählt aus der Gruppe der Enniatine, insbesondere Enniatin A, Enniatin A1, Enniatin B und Enniatin B1; Beauvericin und Apicidin insbesondere vollständig zu eliminieren bzw. so weit zu reduzierten, dass eine schädliche Wirkung auf einen tierischen oder auch menschlichen Organismus nicht zu erwarten ist.

Eine exakte Dosierung der Glycyrrhiza-Pflanzenpräparate und somit die Gewährleistung einer weitgehend vollständigen Reduktion der toxischen Wirkung der Polypeptid-Fungitoxine, nämlich der Enniatine, von Apicidin und von Beauvericin gelingt, wenn, wie dies einer Weiterbildung der Erfindung entspricht, der wässriger Trockenextrakt insbesondere aus den Wurzeln der Glycyrrhiza glabra-Pflanze mit einer Glycyrrhizinsäure zwischen 4 % (w/w) und 10% (w/w), insbesondere 7 % (w/w) in einer Menge von wenigstens 1 g, bevorzugt zwischen 1 g und 100 g, insbesondere bevorzugt zwischen 7 und 50 g für Masthühner, insbesondere bevorzugt zwischen 5 g und 40 g für Legehühner und insbesondere bevorzugt zwischen 5 g und 30 g für Schweine insbesondere Aufzuchtferkel, pro Tonne agrarischem Produkt, insbesondere Futter- oder Nahrungsmittel oder eine äquivalente Menge von einem der anderen Glycyrrhiza-Pflanzenpräparat gemäß der vorliegenden Erfindung eingesetzt wird.

Die Äquivalenz bezieht sich hierbei auf die Konzentration der enthaltenen phytogenen Stoffe bzw. Substanzen in den Glycyrrhiza-Pflanzenpräparaten. Eine gut charakterisierte und leicht quantifizierbare Substanz ist die in den Glycyrrhiza-Wurzeln der vorkommende Glycyrrhizinsäure. Der Anteil der Glycyrrhizinsäure in den Glycyrrhiza-Pflanzenpräparaten dient als Indikator für die Konzentration der Gesamtheit der phytogenen Wirkstoffe bzw. Wirksubstanzen.

Unter einer äquivalenten Menge von Glycyrrhiza-Pflanzenpräparaten werden hierin diese Mengen verstanden, bei denen die Gesamtmenge an Glycyrrhizinsäure gleich ist. So sind zum Beispiel 100 g eines Trockenextrakts mit 4 % (w/w) Glycyrrhizinsäure äquivalent zu 50 g eines Trockenextrakts mit 8% (w/w) Glycyrrhizinsäure. Wird zum Beispiel in einem Futtermittel pro Tonne 50 g eines wässrigen Trockenextrakts mit 10 % (w/w) Glycyrrhizinsäure eingesetzt, so ist dazu das Verabreichen von 100 g eines anderen Glycyrrhiza-Pflanzenpräparats, insbesondere eines anderen wässrigen Trockenextrakts, mit einem Gehalt von 5% (w/w) Glycyrrhizinsäure äquivalent.

Die Wirksamkeit der erfinderischen Glycyrrhiza-Pflanzenpräparate bzw. der Glycyrrhiza-Antidote oder der Antidote, insbesondere deren positive Effekte auf die Leistungsparameter von Nutztieren welche unter Polypeptid-Fungitoxin-Toxikosen leiden, nimmt mit einer steigender Einsatzmenge der Glycyrrhiza-Pflanzenpräparate bzw. der Glycyrrhiza-Antidote oder der Antidote zu und für Polypeptid-Fungitoxinen Konzentrationen im Nahrungs- oder Futtermittel von 34 ppb Enniatin A, 40 ppb Enniatin A1, 510 ppb Enniatin B, 392 ppb Enniatin B1, 4 ppb Enniatin B2, 0,34 ppb Enniatin B3, 717 ppb Beauvericin und 122 ppb Apicidin sind bereits deutliche positive Effekte auf die Leistungsparameter, insbesondere der Gewichtszunahme und der Eierlegerate und somit ein gute Wirksamkeit der Glycyrrhiza-Pflanzenpräparate bzw. der Glycyrrhiza-Antidote oder der Antidote gegen Polypeptid-Fungitoxin-Toxikosen erkennbar. Wobei eine optimale Einsatzmenge der Glycyrrhiza-Pflanzenpräparate bzw. der Glycyrrhiza-Antidote oder der Antidote immer mit der Konzentration der Polypeptid-Fungitoxine korreliert. Je mehr Polypeptid-Fungitoxine im Nahrungs- oder Futtermittel vorhanden sind, desto größer müssen die eingesetzten Mengen der Glycyrrhiza-Pflanzenpräparate bzw. der Glycyrrhiza-Antidote oder der Antidote sein.

Besonders gute Ergebnisse werden erzielt, wenn die Glycyrrhiza-Pflanzenpräparate so eingesetzt werden, dass das agrarische Produkt aus Nahrungs- oder Futtermitteln bestehend aus oder enthaltend wenigstens ein mit wenigstens einem Polypeptid-Fungitoxinen kontaminiertes Erzeugnis gewählt aus der Gruppe Getreide, Mais, Reis, Soja und andere Leguminosen, Raps, Gräser, Kräuter gewählt wird.

Indem, wie dies einer Weiterbildung der Erfindung entspricht, die Glycyrrhiza-Pflanzenpräparate so verwendet werden, dass der weiterhin enthaltene Hilfsstoff aus der Gruppe inerte Träger, Vitamine, Mineralstoffe, phytogene Substanzen, Enzyme und weitere Komponenten zur Detoxifizierung von Mykotoxinen, wie Mykotoxin-abbauende Enzyme, insbesondere Aflatoxin-Oxidasen, Ergotamin-Hydrolasen, Ergotamin-Amidasen, Zearalenon-Esterasen, Zearalenon-Lactonasen, Zearalenon-Hydrolasen, Ochratoxin-Amidasen, Fumonisin-Aminotransferasen, Fumonisin-Carboxyltransferasen, Aminopolyol-Aminoxidasen, Deoxynivalenol-Epoxidhydrolasen, Deoxynivalenol-Dehydrogenasen, Deoxynivalenol-Oxidasen, Trichothecene-Dehydrogenasen, Trichothecene-Oxidasen; und Mykotoxin-transformierende Mikroorganismen, wie z.B. DSM 11798; und Mykotoxinbindende Stoffe, beispielsweise mikrobielle Zellwände oder anorganische Materialien, wie Bentonite gewählt ist, gelingt es neben den Polypeptid-Fungitoxinen Enniatine, Beauvericin und Apicidin auch weitere, teilweise auch in größeren Mengen vorkommende Polypeptid-Fungitoxine abzubauen bzw. unschädlich zu machen.

Neben der Verwendung von Glycyrrhiza-Pflanzenpräparaten zur Reduktion der toxischen Wirkung von Polypeptid-Fungitoxinen in Nahrungs- oder Futtermitteln besteht überdies das Erfordernis nach einem Produkt welches die nachteiligen Wirkungen von derartigen Polypeptid-Fungitoxinen bei einer möglichen Aufnahme derselben, entgegenwirkt.

Zur Lösung dieser Aufgabe ist die vorliegende Erfindung weiterhin auf ein Antidot zur oralen Einnahme zur Reduktion der toxischen Wirkung von wenigstens einem Pölypeptid-Fungitoxin enthaltend wenigstens ein Glycyrrhiza-Pflanzenpräparat gegebenenfalls gemeinsam mit wenigstens einem Hilfsstoff gerichtet. Durch die orale Aufnahme von wenigstens einem Glycyrrhiza-Pflanzenpräparat gegebenenfalls mit wenigstens einem weiteren Hilfsstoff als Antidot gelingt es, die nachteiligen Wirkungen von Polypeptid-Fungitoxinen gleichzeitig mit ihrer Aufnahme zu neutralisieren bzw. zu hemmen bzw. dieser nachteiligen Wirkung vorzubeugen.

Für eine besonders effiziente Wirkung des Antidots kann hierbei, wie dies einer Weiterbildung der Erfindung entspricht, das wenigstens eine Polypeptid-Fungitoxin aus der Gruppe der Enniatine, insbesondere Enniatin A, Enniatin A1, Enniatin B, Enniatin B1, Enniatin B2 oder Enniatin B3; Beauvericin und Apicidin gewählt sein. Dies ist insbesondere deshalb von Bedeutung, da beispielsweise von Enniatinen gezeigt wurde, dass diese mit Proteinen wechselwirken und beispielsweise in Rattenlebermikrosomen die Cholesterinacyltransferase hemmten. Darüber hinaus wurde festgestellt, dass in bis zu 76% aller unverarbeiteten Körner, gewählt aus Getreide, Mais oder Reis Enniatine enthalten sind, so dass diese einen wesentlichen Anteil bei nachteiligen Effekten die durch Polypeptid-Fungitoxine ausübt werden, besitzen. Auch Beauvericin wurde in Bezug auf seine toxische Wirkung bei Mäusen und Geflügel untersucht und festgestellt, dass sich Beauvericin in den Eiern von Geflügel akkumulieren kann. Durch die Aufnahme von kontaminiertem Futter verringert sich bei Geflügel, insbesondere bei Legehühnern in der Folge das Eiergewicht, erniedrigt sich die Eierlegerate und bei Nutztieren im Allgemeinen erhöht sich die Futterumwandlungsrate. Auch Beauvericin wurde in bis zu 54% der nicht behandelten Körner nachgewiesen.

Eine besonders vorteilhafte Wirkung zeigt das Antidot, wenn dies gemäß einer Weiterbildung der Erfindung das Glycyrrhiza-Pflanzenpräparat gewählt ist aus der Gruppe Mehl, wässriger Extrakt, wässriger/ethanolischer Extrakt, wässriger Trockenextrakt und wässriger/ethanolischer Trockenextrakt aus einer gesamten Glycyrrhiza-Pflanze oder aus Wurzeln der Glycyrrhiza-Pflanze, gegebenenfalls gemeinsam mit wenigstens einem Hilfsstoff. In einem derartigen Fall kann es gemeinsam mit dem gegebenenfalls kontaminierten Nahrungs- und Futtermittel aufgenommen werden, wodurch jegliche schädliche Wirkung aus dem Organismus unmittelbar verhindert werden kann.

Eine besonders vorteilhafte und insbesondere gleichmäßig gute Wirkung zeigt das Antidot, wenn die Glycyrrhiza-Pflanze aus der Gruppe Glycyrrhiza glabra und Glycyrrhiza uralensis gewählt ist.

Indem, wie dies einer Weiterbildung der Erfindung entspricht, der wässrige Trockenextrakt enthaltend 4% (w/w) bis 10% (w/w), insbesondere 7% (w/w) Glycyrrhizinsäure eingesetzt wird, konnte in Nahrungs- oder Futtermitteln enthaltene Polypeptid-Fungitoxinen bei ihrem Verzehr vollständig unschädlich gemacht werden, ohne dass die aus der Literatur bekannten ungünstigen Wirkungen von Glycyrrhizinsäure, nämlich Symptome wie Bluthochdruck oder Wassereinlagerungen hintangehalten werden können.

Gemäß einer Weiterbildung der Erfindung wird bevorzugt so vorgegangen, dass wenigstens 1 g des wässrigen Trockenextrakts mit einer Glycerrhizinsäurekonzentration von 7 (w/w), bevorzugt zwischen 1 g und 100 g, insbesondere bevorzugt zwischen 7 g und 50 g für Masthühner, insbesondere bevorzugt zwischen 5 g und 40 g für Legehühner und insbesondere bevorzugt zwischen 5 g und 30 g für Schweine, insbesondere Aufzuchtferkel, oder äquivalente Mengen pro Tonne agrarischem Produkt, insbesondere Futter- oder Nahrungsmittel, eingesetzt sind. Mit einem derartigen Einsatz gelingt es, die Polypeptid-Fungitoxine Enniatin, Beauvericin und Apicidin nahezu vollständig in Nahrungs- oder Futtermitteln unschädlich zu machen, so dass das Auftreten von nachteiligen Effekten, wie z.B. von Vergiftungserscheinungen bei Mensch und Tier mit Sicherheit hintangehalten werden kann bzw. auch diesen nachteiligen Effekten vorgebeugt werden kann.

Indem, wie dies einer Weiterbildung der Erfindung entspricht, das Antidot so weitergebildet ist, dass es mehr als 50% (w/w), bevorzugt mehr als 90 % (w/w), insbesondere 100 % (w/w) des wenigstens einen Glycyrrhiza-Pflanzenpräparats enthält, gelingt es, die synergistische Wirkung der Inhaltsstoffe der Glycyrrhiza-Pflanzenpräparate auszunutzen. Hierbei hat sich überraschenderweise herausgestellt, dass die bekannteste Komponente der Glycyrrhiza-Pflanzenpräparate, nämlich die Glycyrrhizinsäure selbst keine Wirkung gegen Polypeptid-Fungitoxine besitzt. Die positive Wirkung der erfinderischen Glycyrrhiza-Pflanzenpräparate gegenüber Polypeptid-Fungitoxinen basiert daher auf andere derzeit noch nicht näher bekannte Inhaltsstoffe der Süßholzwurzel bzw. in ihrer Wirkung nicht näher bekannten Inhaltsstoffen der Süßholzwurzel, die zur gewünschten Wirkung beitragen, insbesondere dem Verhindern der nachteiligen Effekte der Polypeptid-Fungitoxine.

Indem das Antidot so weitergebildet ist, dass der wenigstens eine Hilfsstoff aus der Gruppe inerter Träger, Vitamine, Mineralstoffe, phytogene Substanzen, Enzyme und weitere Komponenten zur Detoxifizierung von Mykotoxinen, wie Mykotoxin-abbauende Enzyme, insbesondere Aflatoxin-Oxidasen, Ergotamin-Hydrolasen, Ergotamin-Amidasen, Zearalenon-Esterasen, Zearalenon-Lactonasen, Zearalenon-Hydrolasen, Ochratoxin-Amidasen, Fumonisin-Aminotransferasen, Fumionisin-Carboxyltransferasen, Aminopolyol-Aminoxidasen, Deoxynivalenol-Epoxidhydrolasen, Deoxynivalenol-Dehydrogenasen, Deoxynivalenol-Oxidasen, Trichothecene-Dehydrogenasen, Trichothecene-Oxidasen; und Mykotoxin-transformierende Mikroorganismen, wie z.B. DSM 11798; und Mykotoxinbindende Stoffe, beispielsweise mikrobielle Zellwände oder anorganische Materialien, wie Bentonite gewählt ist, gelingt es, die positive Wirkung auch auf weitere potentiell schädliche Substanzen, wie Mykotoxine aus der Gruppe der, Fumonisine, Aflatoxine, Deoxynivalenol, Trichothecene oder Ochratoxine auszudehnen,

In bevorzugter Weise kann hierbei so vorgegangen werden, dass das Antidot zur Herstellung eines Präparats zur Prophylaxe oder Behandlung von Polypeptid-Fungitoxin-Toxikosen herangezogen wird. Insbesondere kann ein.derartiges Präparat zur Prophylaxe und Behandlung der Polypeptid-Fungitoxin-Toxikosen versursacht durch Polypeptid-Fungitoxine gewählt aus der Gruppe der Enniatine, insbesondere Enniatin A, Enniatin A1, Enniatin B, Enniatin B1, Enniatin B2 oder Enniatin B3; Beauvericin und Apicidin herangezogen werden. Hierbei gelingt es insbesondere, die schädlichen Wirkungen von Polypeptid-Fungitoxinen vorzubeugen bzw. zu beheben, ohne dass gleichzeitig nachteilige Effekt verursacht durch Glycyrrhizinsäure, wie Bluthochdruck oder dgl. auftreten.

Die Erfindung zielt weiterhin auf ein Verfahren zur akuten Behandlung eines Subjekts welches an einer Polypeptid-Fungitoxin-Toxikose leidet und/oder zur prophylaktischen Behandlung eines Subjekts zur Vorbeugung einer Polypeptid-Fungitoxin-Toxikose durch orale Verabreichung einer effektiven Menge eines Glycyrrhiza-Antidots an das Subjekt ab. Mit einem derartigen Verfahren gelingt es die bis dato wenig erforschten, aber weit verbreiteten Polypeptid-Fungitoxin-Toxikosen, insbesondere durch Enniatine, wie Enniatin A, Enniatin A1, Enniatin B, Enniatin B1, Enniatin B2 oder Enniatin B3; Beauvericin und Apicidin verursachte Polypeptid-Fungitoxin-Toxikosen zu behandeln oder ihnen vorzubeugen, indem als Glycyrrhiza-Antidots eingesetzt wird.

Als Subjekte werden Menschen und Tiere verstanden, jedoch insbesondere Nutztiere, bevorzugt Schweine sowie Geflügel wie Masthühner oder Legehühner, Truthähne, Rinder oder Kälber.

Polypeptid-Fungitoxin-Toxikosen können vor allem durch verunreinigte, d.h. mit Polypeptid-Fungitoxinen kontaminierte Futter- oder Nahrungsmittel verursacht werden, wobei bereits durch Mengen von etwa 500 ppb an Polypeptid-Fungitoxinen toxische Wirkungen auftreten. Eine Polypeptid-Fungitoxin-Toxikose wird hierin insbesondere bei Nutztieren als Krankheit definiert, die durch Polypeptid-Fungitoxinen ausgelöst wird und zu einer Verschlechterung von wenigstens einem Leistungsparameter von wenigstens 4 %, bevorzugt 10 % im Vergleich zur positiven Kontrollgruppe führt.

Bei Masthühnern treten mindestens ab einer Polypeptid-Fungitoxin Gesamtkonzentration von etwa 5000 ppb, insbesondere 4986,34 ppb, klare äußere Anzeichen einer Polypeptid-Fungitoxin-Toxikose auf, insbesondere Verschlechterungen von Leistungsparametern.

Bei Legehühner treten mindestens ab einer Polypeptid-Fungitoxin Gesamtkonzentration von etwa 2000 ppb, insbesondere 1985 ppb, klare äußere Anzeichen einer Polypeptid-Fungitoxin-Toxikose auf, insbesondere Verschlechterungen von Leistungsparametern.

Bei Schweinen, vor allem bei Aufzuchtferkel, treten mindestens ab einer Polypeptid-Fungitoxin Gesamtkonzentration von etwa 7000 ppb, insbesondere 7183,6 ppb, klare äußere Anzeichen einer Polypeptid-Fungitoxin-Toxikose auf, insbesondere Verschlechterungen von Leistungsparametern.

Als Glycyrrhiza-Antidots zur Behandlung und/oder Prophylaxe der Polypeptid-Fungitoxin-Toxikosen kann jegliches oben beschriebene Antidot bzw. Glycyrrhiza-Pflanzenpräparat eingesetzt werden, wobei ein wässriger Trockenextrakt aus der Glycyrrhiza glabra Wurzel bevorzugt ist.

Die effektive Menge des Glycyrrhiza-Antidots hängt von der Menge der Polypeptid-Fungitoxine im kontaminierten Futter- oder Nahrungsmittel ab und kann auch von der Art des Subjekts abhängen. Die effektive Menge eines Glycyrrhiza-Antidots welches ein 100%iger wässriger Trockenextrakts aus der Glycyrrhiza glabra Wurzel mit einer Glycyrrhizinsäurekonzentration des von 7 % (w/w) ist, beträgt in etwa pro Kilogramm Futter- oder Nahrungsmittel

- für Masthühner wenigstens 1 mg, 1 mg bis 100 mg, bevorzugt 7 mg bis 50 mg.
- für Legehühner wenigstens 1 mg, 1 mg bis 100 mg, bevorzugt 5 mg bis 40 mg.
- für Schweine, insbesondere Aufzuchtferkel, wenigstens 1 mg, 1 mg bis 100 mg, bevorzugt 5 mg bis 40 mg.

Dem Fachmann ist klar, dass anstatt des 100%igen wässrigen Trockenextrakts aus der Glycyrrhiza glabra Wurzel mit einer Glycyrrhizinsäurekonzentration von 7% (w/w) auch jegliches andere hierin beschriebene Glycyrrhiza-Antidots verwendet werden kann, solange dieses in einer äquivalenten Menge eingesetzt wird.

Diese effektive Menge ist ausreichend um die toxische Wirkung der Polypeptid-Fungitoxine nahezu vollständig aufzuheben, wodurch die negative Auswirkung der Polypeptid-Fungitoxine auf die Gesundheit der Subjekte und auf deren Leistungsparameter nahezu aufgehoben wird.

Die Erfindung wird nachfolgend anhand von Ausführungsbeispielen näher erläutert.

Beispiel 1: Herstellung der Glycyrrhiza-Pflanzenpräparate

Bei den nachfolgenden Versuchen bzw. Beispielen wurde als Glycyrrhiza-Pflanzenpräparat ein sprühgetrockneter Trockenextrakt, welcher als Endprodukt 7 % (w/w) Glycyrrhizinsäure und 0,003 % (w/w) Glycyrrhetinsäure enthält, eingesetzt. Wobei der Gehalt an Glycyrrhizinsäure und Glycyrrhetinsäure, wie dem Fachmann bekannt ist, je nach Ausgangsmaterial und Prozessführung variieren kann. Solche Glycyrrhiza-Pflanzenpräparate mit abweichenden Konzentrationen von Glycyrrhizinsäure und Glycyrrhetinsäure sind von der Erfindung ebenfalls mitumfasst, wobei deren Anwendungsmenge je nach Gehalt der phytogenen Inhaltsstoffe insofern angepasst werden müssen, sodass jeweils äquivalente Mengen, bezogen auf den Glycyrrhizinsäuregehalt, eingesetzt werden.

Für die Herstellung dieses Extrakts wurde wie folgt vorgegangen: Nach der Ernte der 3 bis 4 Jahre alten Glycyrrhiza glabra Wurzel wurde diese zuerst zerkleinert und mit Wasser zu einem feinen Brei zerrieben, der über einige Stunden, insbesondere 3 Stunden, eingekocht und konzentriert wird. Der Auszug wird nach dem Kolieren und Absitzen lassen mittels einer wässrigen Dampfextraktion mit vermindertem Druck weiter extrahiert. Dabei entsteht ein natürlicher, konzentrierter Saft, der anschließend unter ständigem Rühren weiter gekocht und noch weiter aufkonzentriert wird, wodurch der wässrige Glycyrrhiza Extrakt entsteht.

Als weiterer Prozess- und Trocknungsschritt wird der wässrige Glycyrrhiza Extrakt sprühgetrocknet. Dieses Schnellverdampfungsverfahren beruht auf der Trocknung kleiner Tröpfchen in einem temperierten inerten Trocknungsgas. Dabei wird die flüssige Formulierung in feine Tröpfchen zerstäubt und gleichmäßig in der Trocknungsluft des Sprühturms verteilt. Das führt zu einer Vergrößerung der Gesamtoberfläche der Flüssigkeit, wodurch das Produkt in kurzer Zeit getrocknet werden kann. Als Endprodukt enthält man ein trockenes, frei fließendes Pulver mit relativ gleichmäßiger Partikelgröße, den Glycyrrhiza-Trockenextrakt. Anschließend wird das Produkt qualitativ überprüft und kann durch die orale Aufnahme von Subjekten oder für die Durchführung weiterer Versuche wieder in Lösung gebracht werden (Karasaaslaan und Dalgici, J. Food Sci. Technol., 2014, 51(11), 3014ff; Bauer et al., Lehrbuch der Pharmazeutischen Technologie, 2012, , ISBN 978-3-8047-2552-2).

Beispiel 2: *In-vitro* protektiver Effekt des Glycyrrhiza-Trockenextrakts gegenüber Polypeptid-Fungitoxinen

Um die Zusammenhänge von Polypeptid-Fungitoxinen an epithelialen Gewebe unter kontrollierten Bedingungen zu testen, wurden *in vitro* Studien durchgeführt. Hierbei wurde von in der Literatur beschriebenen und gut charakterisierten Testsystemen auf Basis von Zellkultur Gebrauch gemacht. Für Futtermitteladditive werden Epithelzellen aus dem betreffenden Organ, z.B. in diesem Fall dem Schweinedarm IPEC-J2 Zellen, bevorzugt. Die verwendeten Schweinedarmepithelzellen haben den Vorteil, dass es sich um nichttransformierte Zellen handelt, die noch alle wichtigen Eigenschaften normaler Darmzellen haben und große physiologische Ähnlichkeiten, wie z.B. die Ausbildung funktionsfähiger Tight Junction Proteine, die vor allem für die Darmbarriere essentiell sind, sowie die Expression charakteristischer Enzyme und Transportsysteme (z.B. P-Glykoprotein, Cytochrom P450 3A4, Vit. B 12 Transporter u.a.).

Solche *in vitro* Testsysteme sind wichtige und anerkannte Verfahren um *in vivo* Ergebnisse vorherzusagen und sogar einen Verzicht von Bioverfügbarkeits-Studien möglich machen. Eine humane Zelllinie, Caco-2 Zellen, die den IPEC-J2 Zellen in vieler Hinsicht sehr ähnlich ist, wurde zur Untersuchung des Transports von Arzneistoffen bereits Ende der 1980er Jahre publiziert. In der folgenden Zeit wurden gute Korrelationen zwischen den aus den Zellmonolayern gewonnenen Permeabilitätsdaten und der oralen Absorptionsquote gefunden. Dadurch hat dieses Modell in der pharmazeutischen Industrie Eingang gefunden. Weiterhin wurde von der amerikanischen Zulassungsbehörde FDA hierzu eine Richtlinie publiziert, in welcher ein Zusammenhang mit dem sogenannten "Biopharmaceutics Classification System (BCS)" die Bestimmung der intestinalen Permeabilität anhand von validierten Zellkultursystemen vorgesehen ist. Auf der Grundlage solcher *in vitro* Daten ist in bestimmten Fällen ein Verzicht auf Bioverfügbarkeits-Studien möglich.

Für die *in vitro* Untersuchungen der Glycyrrhiza-Trockenextrakte wurde dieser abermals extrahiert, wobei ein sekundärer Glycyrrhiza Extrakt hergestellt wurde. Der sekundäre Glycyrrhiza Extrakt wurde hergestellt, indem jeweils 1 g des Glycyrrhiza-Trockenextrakts und 9 ml 70% Ethanol eingewogen, gemischt und bei Raumtemperatur für eine Stunde bei 700 U/min geschüttelt wurden. Danach wurde die Lösung abzentrifugiert und der flüssige Überstand mit einem 0,2 $\mu$m Filter steril filtriert und weiter mit IPEC-J2 Medium verdünnt. Der sekundäre Glycyrrhiza Extrakt wurde *in vitro* in folgenden Konzentrationen getestet: 250 $\mu$g, 500 $\mu$g und 1.000 $\mu$g Glycyrrhiza-Trockenextrakt pro ml Medium im Versuchsansatz, im Folgenden auch als 250, 500 und 1.000 $\mu$g/ml sekundärer Glycyrrhiza Extrakt bezeichnet.

Die Tatsache, dass ethanolische Extrakte mehr Inhaltsstoffe lösen können als reine wässrige Extrakte und somit näher an der *in vivo* Situation sind, bei der durch das saures bzw. basisches Milieu sowie diverser Enzyme im Münd-Magen-Darm Trakt ebenfalls mehr Inhaltsstoffe als bei rein wässriger Extraktion in Lösung gehen, war der ausschlaggebende Grund warum für die *in vitro* Versuche sekundäre Extrakte mit 70% Ethanol hergestellt wurden.

Als einer der phytogenen Hauptbestandteile der Süßholzwurzel wird in der Literatur die Glycyrrhizinsäure betrachtet. Die Glycyrrhizinsäure und Glycyrrhetinsäure Konzentrationen in den hergestellten sekundären Glycyrrhiza Extrakten wurden mittels LC-MS/MS (Agilent 1290 Infinity and Sciex 5500 QTrap) bestimmt. Dafür wurden diese Extrakte mit jeweils mit einer Kinetex Biphenyl (100 x 3 mm) Säule aufgetrennt. Multiple Reaction Monitoring (MRM) war bei 471/105 und 471/119 Da für Glycyrrhetinsäure bzw. 823/453 und 823/647 Da für Glycyrrhizinsäure. Aufgrund der guten Löslichkeit der Glycyrrhizinsäure und der Glycyrrhetinsäure kann davon ausgegangen werden, dass die gesamte im wässrigen Trockenextrakt enthaltende Menge auch im sekundären Extrakt gelöst ist. Die hergestellten Glycyrrhiza-Trockenextrakte enthielten 7 % (w/w) Glycyrrhizinsäure und 0,03 % (w/w) Glycyrrhetinsäure.

Die Effektivität der Verabreichungsform *in vivo* ist immer von der Bioverfügbarkeit der Substanz und dadurch auch von einem biokinetischen Prozess abhängig - das trifft bei *in vitro* Experimenten in der Regel nicht zu. Aus diesem Grund müssen bei *in vitro* Experimenten häufig höhere Konzentrationen eingesetzt werden, um dieselben Effekte wie *in vivo* sehen zu können (Gülden und Seibert, ALTEX 22, Special Issue 2, 2005). Die in den vorliegenden *in vitro* Versuchen verwendeten Konzentrationen von 250 $\mu$g, 500 $\mu$g und 1.000 $\mu$g Glycyrrhiza-Trockenextrakt pro ml Medium entsprechen *in vivo* Konzentrationen von in etwa 250 g bis 1 kg Glycyrrhiza-Trockenextrakt pro Tonne Futter- oder Nahrungsmittel. Dies ist um den Faktor 33 bis 50 mehr als die 5 g/t bis 30 g/t die in den *in vivo* Versuchen (siehe Beispiel 3 bis 5) eingesetzt wurden.

Die von Geens und Niewold (Cytotechnology, 2011, 63, 415ff) beschriebene TEER Methode wurde in adaptierter Form eingesetzt. Bei diesem in vitro Modell werden intestinale porzine Epithelzellen (IPEC-J2, DSMZ Nr.: ACC 701, Passage 1-15) in einem "Insert" auf einer permeablen Polyester-Membran (1,12 cm$^2$ Fläche, 0,4 $\mu$m Porengröße, 12 mm Membrandurchmesser) kultiviert und 8 Tage lang bei 39 °C und 5% $CO_2$ im Brutschrank differenziert. Pro Insert werden 1 x 10^5 Zellen in 0,5 ml Medium auf die jeweilige Membran aufgetragen. Während dieser Differenzierungszeit wurde alle 2 Tage das verbrauchte Medium abgesaugt und mit frischem Medium ersetzt. Dieses Insert war in einer 12-well Zellkulturplatte befestigt und die Zellen wurden von unten (basolaterales Kompartiment) mit 1,5 ml Medium sowie von oben (apikales Kompartiment) mit 0,5 ml Medium versorgt. Diese Kompartimente spiegeln die Darm- (apikal "A") und die Blutseite (basolateral "B") wieder. Bei dem Medium handelt es sich um ein DMEM/Ham's F12 (1:1) Medium erweitert mit 1% IST, 2,5 mM Glutamax, 16 mM Hepes, 6 ng/ml EGF, 5% fetales Kälberserum und 100 IU/ml Penicillin und 100 µg/ml Streptomycin.

Differenzierte Zellen bilden eine Zellbarriere, die mit "Tight Junction" Proteine untereinander stark verbunden sind. Des Weiteren sind diese Zellen wie im Schweinedarm polarisiert ausgerichtet und können als repräsentatives Modell verwendet werden. Da die Darmbarriere die erste Abwehrlinie im Verdauungstrakt gegen Pathogene und Giftstoffe darstellt, ist es für die Gesundheit wichtig, dass diese unbeschädigt ist. Die Intaktheit der Zellbarriere wird mit Hilfe eines Voltohmmeters gemessen: Der elektrische Widerstand zwischen den zwei Kompartimenten wird als TEER (transepithelial electrical resistance)-Wert bezeichnet und in kOhm x cm$^2$ dargestellt. Vor der Messung muss das Voltohmmeter auf "Power" und "R" gestellt werden. Die längere Elektrode wird dann in das basolaterale Kompartiment und die kürzere Elektrode in das apikale Kompartiment eingeführt. Der Zelllayer darf dabei nicht berührt werden.

Der sekundäre Glycyrrhiza Extrakt mit einer Konzentration von 100 mg/ml wird mit Medium auf die gewünschte Konzentration verdünnt (250 µg/ml, 500 µg/ml oder 1.000 µg/ml). Das verbrauchte Medium im apikalen Kompartiment wird abgesaugt und mit 250 µl Toxin sowie 250 µl Süßholzextrakt (beides 2-fach im Medium konzentriert) aufgefüllt und für weitere 72 h bei 39 °C und 5 % $CO_2$ inkubiert. Nach 24 h, 48 h und 72 h wird die TEER-Messung durchgeführt. Alle Messungen erfolgten mindesten in 3 Replikaten, von denen die Mittelwerte für die weiteren Berechnungen herangezogen wurden.

Tabelle 1: Zeitlicher Ablauf des TEER-Zellkulturversuchs

| Tag 0 | Zellen in die Inserts aussäen |
|---|---|
| Tag 2, 4, 7 | Medium erneuern |
| Tag 8 | Zugabe der Toxine (Negativkontrolle); Zugabe der Glycyrrhiza Extrakte (Glycyrrhiza Kontrolle); Keine Zugabe (unbehandelte Zellen; Zellkontrolle); Zugabe der Toxine und des Glycyrrhiza Extrakts; Zugabe von Glycyrrhizinsäure |
| Tag 9, 10, 11 | TEER-Wert Messung |
| Tag 11 | Zytotoxizitätstest |

Der TEER-Wert war bei den unbehandelten Zellen von Tag 8 bis Tag 11 konstant (Zellkontrolle), ebenso nach der Zugabe des sekundären Glycyrrhiza Extrakts in den Konzentrationen von 250 µg/m, 500 µg/m oder 1.000 µg/ml (Glycyrrhiza Kontrolle) am Tag 8.

Durch die Zugabe der Polypeptid-Fungitoxine Beauvericin, Enniatine und Apicidin in unterschiedlichen Konzentrationen (0,2 - 10 µM) (Negativkontrolle) am Tag 8, wurde der TEER-Wert allerdings gesenkt, da die Toxine die Darmbarriere schädigen.

Die zeitgleiche Zugabe der Polypeptid-Funigtoxine in den Konzentrationen 0,438 µM Apicidin, 5 µM für Enniatin A, B, B1, Beauvericin sowie 10 µM Enniatin A1 jeweils gemeinsam mit dem sekundären Glycyrrhiza Extrakt in den Konzentrationen 250 µg/ml, 500 µg/ml oder 1.000 µg/ml zeigte eine deutlich geringere bzw. gar keine Abnahme des TEER-Werts im Vergleich zur entsprechenden Negativkontrolle. Die Hinzugabe des sekundäre Glycyrrhiza Extrakts konnte somit den negativen Effekten der Polypeptid-Fungitoxine entgegenwirken (siehe Tabelle 2). Die Berechnung der protektiven Effekte der sekundäre Glycyrrhiza Extrakte gegenüber den Polypeptid-Fungitoxinen erfolgte anhand folgender Formel:

$$\text{Protektiver Effekt (\%)} = (\text{TEER Mittelwert (Toxin + Extrakt)}/\text{TEER Mittelwert (Toxin)} \times 100) - 100$$

Zum Beispiel für Enniatin A [5 µM] nach 24 Stunden inkubiert mit 1.000 µg/ml sekundärer Glycyrrhiza Extrakt:

TEER Mittelwert Enniatin A: 3,928 kOhm x cm$^2$
TEER Mittelwert Enniatin A + sekundäre Glycyrrhiza Extrakt: 6,451 kOhm x cm$^2$ Protektiver Effekt: (6,451 kOhm x cm$^2$/ 3,928 kOhm x cm$^2$) x 100) - 100 = 64,2 %

Nach der letzten TEER-Messung am Tag 11 wird ein Zytotoxizitätstest (Neutralrot-Test) durchgeführt, um einen zytotoxischen Effekt der getesteten Toxin- und Süßholzextrakt-Konzentrationen auszuschließen. Es wurden ausschließlich Versuche im nicht zytotoxischen Konzentrationsbereich mit einer Zell-Viabilität über 99% durchgeführt.

Tabelle 2: Protektiver Effekt von 250 µg/ml, 500 µg/ml und 1.000 µg/ml sekundärem Glycyrrhiza Extrakt gegen Enniatin A, A1, B, B1, Beauvericin und Apicidin zu drei verschiedenen Messzeitpunkten (24, 48 und 72 Stunden entspricht Tag 9, 10 und 11).

| Toxin | ENN A 5 µM | ENN A1 10 µM | ENN B 5 µM | ENN B1 5 µM | BEA 5 µM | API 0,438 µM |
|---|---|---|---|---|---|---|
| Zeitpunkt | 250 µg/ml sekundäre Glycyrrhiza Extrakt | | | | | |
| 24 h | 11,8 | 2,6 | 6,1 | 2,9 | 12,8 | 2,1 |
| 48 h | 34,8 | 11,6 | 4,8 | 3,6 | 2,0 | 3,0 |
| 72 h | 28,5 | 6,8 | 4,6 | 3,5 | 1,5 | 7,7 |
| | | | | | | |
| | 500 µg/ml sekundärer Glycyrrhiza Extrakt | | | | | |
| 24 h | 13,3 | 11,9 | 5,5 | 21,9 | 26,2 | 8,4 |
| 48h | 36,6 | 23,5 | 8,5 | 16,7 | 18,4 | 16,4 |
| 72 h | 41,4 | 20,3 | 15,0 | 27,3 | 27,6 | 19,6 |
| | | | | | | |
| | 1.000 µg/ml sekundärer Glycyrrhiza Extrakt | | | | | |
| 24 h | 64,2 | 28,9 | 15,3 | 31,4 | 0,3 | 42,4 |
| 48 h | 45,8 | 68,5 | 25,0 | 27,5 | 44,7 | 24,0 |
| 72 h | 37,6 | 72,1 | 21,4 | 59,8 | 19,1 | 17,8 |

Die stärksten protektiven Effekte durch sekundären Glycyrrhiza Extrakt waren bei Enniatin A ([1.000 µg/ml] bei 24 h und 48 h, [500 µg/ml] bei 72 h) und A1 ([1.000 µg/ml] bei 48 h und 72 h) sichtbar. Bei allen Toxinen konnte eine konzentrationsabhängige Verbesserung durch sekundäre Glycyrrhiza Extrakte gesehen werden. Auch gegen Enniatin B und B1 war ein guter Effekt zu sehen, wobei sich hier vor allem die höchste Glycyrrhiza Extrakt Konzentration (1.000 µg/ml) positiv auf den TEER-Wert auswirkte. Gegen Beauvericin und Apicidin waren bei 500 µg/ml und 1.000 µg/ml sekundärer Glycyrrhiza Extrakt positive Effekte zu sehen.

Wirkung der Glycyrrhizinsäure

Um eine mögliche Wirkung der Glycyrrhizinsäure gegen Enniatine A, A1, B, B1, Beauvericin und Apicidin zu untersuchen, wurde die Glycyrrhizinsäure anstatt des sekundären Glycyrrhiza Extraktes im TEER Test wie oben beschrieben in einer Konzentration von 70 µg/ml (entspricht 1000 µg/ml sekundärer Glycyrrhiza Extrakt) getestet. Es konnte jedoch kein positiver Effekt gegenüber den Negativkontrollen gezeigt werden. Dies zeigt überraschenderweise ganz klar, dass die positive Wirkung der Glycyrrhiza-Pflanzenpräperate nicht auf dem bekanntesten phytogenen Wirkstoff Glycyrrhiza-Pflanze, nämlich der Glycyrrhizinsäure, beruht.

Bindeversuche

Um den Mechanismus des positiven bzw. protektiven Effekts der Glycyrrhiza-Pflanzenpräparate besser zu verstehen, wurden Bindeversuche mit dem wässrigen Glycyrrhiza-Trockenextrakt und den Polypeptid-Funigtoxinen durchgeführt.

Dafür wurde in einem Glasgefäß 1.000 ml Pufferlösung (1.36 g Natriumacetat Trihydrat und 0.79 g Calciumacetat, pH-Wert = 8,0) mit 100 g Futtermatrix (Ferkelfutter bestehend aus 50% (w/w) Weizen, 10% (w/w) Weizenkleie, 20% (w/w) Soja, 10% (w/w) Gerste, 10% (w/w) Mineralstoffen) vorinkubiert (24 h bei 4 °C) um Adsorptionseffekte der hydrophoben Toxine am Glas zu minimieren. Danach wurden die festen Bestandteile abzentrifugiert und die klare Lösung zu jeweils 50 ml in Glasgefäße überführt. Zu diesen 50 mL wurde jeweils so viel wässriger Glycyrrhiza-Trockenextrakt mit 7 % (w/w) Glycyrrhizinsäure hinzugefügt, dass die Konzentration des Extrakts im Bindeversuchsansatz jeweils 3 mg/l bzw. 3 g/l betrug. Danach wurden die Toxine Enniatin A, A1, B, B1, Beauvericin und Apicidin hinzugegeben, sodass die Konzentration im Bindeversuchsansatz jeweils 100 ppb betrug. Im Anschluss wurden die Binderversuchsansätze bei 37°C unter ständigem Rühren für 24 h inkubiert. Am Anfang sowie nach 24 h wurden Proben entnommen und mittels LC-MS/MS auf Enniatin A, A1, B, B1, Beauvericin und Apicidin wie oben beschrieben analysiert. In keiner getesteten Extraktkonzentration (3 g/l oder 3 mg/l) konnte eine Reduktion einer Toxinkonzentration gemessen werden. Daher wird ausgeschlossen, dass der positive, protektive Effekt der Glycyrrhiza-Pflanzenpräparate durch eine Adsorption bzw. Absorption der Toxine an Bestandteile bzw. durch gelöste Stoffe des Glycyrrhiza-Pflanzenpräparates zustande kommt.

Beispiel 3: Fütterungsversuch mit Masthühnern

Um den Effekt von Glycyrrhiza-Pflanzenpräparaten gegen Polypeptid-Fungitoxine bei Geflügel zu bewerten, wurden Masthühner-Fütterungsversuche mit einem Glycyrrhiza-Antidot als Futtermittelzusatz durchgeführt. Als Glycyrrhiza-Antidot wurde ein 100%iger wässriger Glycyrrhiza-Trockenextrakt aus den Wurzeln der Glycyrrhiza glabra Pflanze mit einer Glycyrrhizinsäurekonzentration von 7% (w/w) eingesetzt. Der wässrige Glycyrrhiza-Trockenextrakt wurde wie in Beispiel 1 beschrieben hergestellt.

Dazu wurden 800 Ross Küken mit einem Startgewicht von 40 g in vier Versuchsgruppen zu je 10 Buchten mit jeweils 20 Küken aufgeteilt. Das Futter wurde *ad libitum* verabreicht.

Die positive Kontrollgruppe erhielt gewöhnliches Hühnerfutter (Phase 1, Tag 0 - 14: Mais 58 %, Soja HP 31,25 %, Prämix BR 5 % Universal 6,25 %, Megafat 1,25 %, Sojaöl 2,50 %, Aminosäuren 0,50 %, Monocalciumphosphat 0,25 %. Phase 2, Tag 15 - 35: Mais 60 %, Soja HP 29,35 %, Prämix BR 5 % Universal 6,00 %, Megafat 2,50 %, Sojaöl 2,00 %, Aminosäuren 0,15 %).

Die negative Kontrollgruppe erhielt mit Polypeptid-Fungitoxinen kontaminiertes Hühnerfutter der gleichen Formulierung wie die positive Kontrollgruppe. Die Gesamtkontaminierung an Polypeptid-Funigtoxine betrug 4986,34 ppb, wobei Beauvericin zu 1197 ppb, Enniatine zu 2763,34 ppb (ENN A 34 ppb, ENN A1 175 ppb, ENN B 1700 ppb, ENN B1 803 ppb, ENN B2 51 ppb, ENN B3 0,34) und Apicidin zu 1026 ppb im finalen Hühnerfutter vorlag. Die natürliche Kontaminierung durch Aflatoxin und Deoxynivalenol war jeweils < 1 ppb und somit vernachlässigbar.

Die zwei Testgruppen erhielten dasselbe mit Polypeptid-Fungitoxinen kontaminierte Futter wie die negative Kontrollgruppe und zusätzlich das Glycyrrhiza-Antidot in einer Einmischrate von 7 g (Testgruppe 1) und 50 g (Testgruppe 2) pro Tonne Futtermittel. Der Versuchszeitraum betrug 35 Tage, wobei die Tiere am Tag 0 und 35 gewogen wurden.

Die Leistungsparameter Lebendgewicht sowie Futterverwertungsrate sind in Tabelle 4 und 5 dargestellt. Die Tiere der negativen Kontrollgruppe litten dabei unter Polypeptid-Fungitoxin-Toxikose, was einen flüssigeren Kot und eine deutliche Reduktion des Lebendgewichts (Verschlechterung um 11,4 %) und eine Verschlechterung der Futterverwertungsrate (Verschlechterung um 4,9 %) im Vergleich zur positiven Kontrollgruppe zur Folge hatte. Die Verabreichung des Glycyrrhiza-Antidots in den beiden Testgruppen verursachte eine Reduktion der toxischen Wirkung der Polypeptid-Fungitoxine und somit eine weit weniger ausgeprägte bzw. nicht mehr vorhandene Polypeptid-Fungitoxin-Toxikose, was in beiden Einmischraten das Lebendgewicht der Masthühner und die Futterverwertungsrate im Vergleich zur negativen Kontrollgruppe deutlich verbesserte.

Das erfinderische Glycyrrhiza-Antidot bzw. Glycyrrhiza-Pflanzenpräparat kann somit zur Reduktion der toxischen Wirkung von wenigstens einem Polypeptid-Fungitoxin in agrarischen Produkten als auch zur Steigerung der Leistungsparameter Lebendgewicht und Futterverwertungsrate von mit Polypeptid-Fungitoxinen kontaminiertem Futter für Nutztiere, insbesondere für Masthühner, verwendet werden sowie zur Behandlung und Prophylaxe von Polypeptid-Fungitoxin-Toxikosen.

Tabelle 4: Wirkung des Glycyrrhiza-Antidots bzw. Glycyrrhiza-Pflanzenpräparat auf das Lebendgewicht

| Tag | Positive Kontrollgruppe [g] | Negative Kontrollgruppe [g] | Testgruppe 1 [g] | Testgruppe - 2 [g] |
|---|---|---|---|---|
| 35 | 1963 | 1739 | 1870 | 1897 |

Tabelle 5: Wirkung von Glycyrrhiza-Antidots bzw. Glycyrrhiza-Pflanzenpräparat auf die Futterverwertungsrate in einem Beobachtungszeitraum von 35 Tagen.

| Tag | Positive Kontrollgruppe [g/g] | Negative Kontrollgruppe [g/g] | Testgruppe 1 [g/g] | Testgruppe 2 [g/g] |
|---|---|---|---|---|
| 1-35 | 1,74 | 1,83 | 1,76 | 1,74 |

Beispiel 4: Fütterungsversuch mit Legehühnern

Um den Effekt von Glycyrrhiza-Pflanzenpräparaten gegen Polypeptid-Fungitoxine bei Geflügel zu bewerten, wurden Legehühner-Fütterungsversuche mit einem Glycyrrhiza-Antidot als Futtermittelzusatz durchgeführt. Als Glycyrrhiza-Antidot wurde ein 100 %iger wässriger Glycyrrhiza-Trockenextrakt aus den Wurzeln der Glycyrrhiza glabra Pflanze der mit einer Glycyrrhizinsäurekonzentration von 7 % (w/w) eingesetzt. Der wässrige Glycyrrhiza-Trockenextrakt wurde wie in Beispiel 1 beschrieben hergestellt.

Dazu wurden 160 Lohmann Brown Legehühner in vier Versuchsgruppen zu je 10 Buchten mit jeweils 4 Hennen aufgeteilt. Das Futter wurde *ad libitum* verabreicht. Der Versuch startete im Alter von 22 Wochen.

Die positive Kontrollgruppe erhielt gewöhnliches Legehennenfutter (Weizen 32,1%, Mais 30,00%, Soja HP 25,00 %, Calciumcarbonat 8,60 %, Legehennenprämix 2,00 %, Rapsöl 1,90%, Biotronic SE forte 0,40 %) (Biotronic ist eine Marke der Erber Aktiengesellschaft).

Die negative Kontrollgruppe erhielt mit Polypeptid-Fungitoxinen kontaminiertes Legehennenfutter der gleichen Formulierung wie die positive Kontrollgruppe. Die Gesamtkontaminierung an Polypeptid-Funigtoxine betrug 1985 ppb, wobei Beauvericin zu 835 ppb, Enniatine zu 1028 ppb (ENN A 35 ppb, ENN A1 76 ppb, ENN B 510 ppb, ENN B1 392 ppb, ENN B2 15 ppb) und Apicidin zu 122 ppb im finalen Legehennenfutter vorlag. Die natürliche Kontaminierung durch Aflatoxin und Deoxynivalenol war jeweils < 1 ppb und somit vernachlässigbar.

Die zwei Testgruppen erhielten dasselbe mit Polypeptid-Fungitoxinen kontaminierte Futter wie die negative Kontrollgruppe und zusätzlich das Glycyrrhiza-Antidot in einer Einmischrate von 5 g (Testgruppe 1) und 40 g (Testgruppe 2) pro Tonne Futtermittel. Der Versuchszeitraum betrug 14 Tage.

Die Leistungsparameter Eilegerate (Prozent der Hühner, die täglich ein Ei legen), durchschnittliches Eigewicht und Futterverwertungsrate wurden während des Versuchszeitraums bestimmt und sind in Tabelle 6 dargestellt. Die Tiere der negativen Kontrollgruppe litten dabei unter Polypeptid-Fungitoxin-Toxikose, was eine flüssigeren Kot und insbesondere eine deutliche Verschlechterung der Leistungsparameter (Eilegerate: Verschlechterung: 6,1 %; Eigewicht: Verschlechterung: 3,6 %; Futterverwertung: Verschlechterung: 7,1 %) zur Folge hatte. Die Verabreichung des Glycyrrhiza-Antidots in den beiden Testgruppen verursachte eine Reduktion der toxischen Wirkung der Polypeptid-Fungitoxine und somit eine weit weniger ausgeprägte bzw. nicht mehr vorhandene Polypeptid-Fungitoxin-Toxikose, was in beiden Einmischraten die Leistungsparameter im Vergleich zur negativen Kontrollgruppe deutlich verbesserte.

Das erfinderische Glycyrrhiza-Antidot bzw. Glycyrrhiza-Pflanzenpräparat kann somit zur Reduktion der toxischen Wirkung von wenigstens einem Polypeptid-Fungitoxin in agrarischen Produkten als auch zur Steigerung der Leistungsparameter Eilegerate, durchschnittliches Eigewicht und Futterverwertungsrate von mit Polypeptid-Fungitoxinen kontaminiertem Futter für Nutztiere, insbesondere bei Legehennen, verwendet werden sowie zur Behandlung und Prophylaxe von Polypeptid-Fungitoxin-Toxikosen.

Tabelle 6: Leistungsparameter

|  | Positive Kontrollgruppe | Negative Kontrollgruppe | Testgruppe 1 | Testgruppe 2 |
|---|---|---|---|---|
| Eilegerate [%] | 99 | 93 | 96 | 98 |
| durchschnittliches Eigewicht [g] | 56 | 54 | 55 | 56 |
| Futterverwertungsrate [g/g] | 1,83 | 1,97 | 1,89 | 1,84 |

Beispiel 5: Fütterungsversuch mit Aufzuchtferkel

Um den Effekt von Glycyrrhiza-Pflanzenpräparaten gegen Polypeptid-Fungitoxine bei Schweinen zu bewerten, wurde ein Aufzuchtferkel-Fütterungsversuch mit einem Glycyrrhiza-Antidot als Futtermittelzusatz durchgeführt. Als Glycyrrhiza-Antidot wurde ein 100 %iger wässriger Glycyrrhiza-Trockenextrakt aus den Wurzeln der Glycyrrhiza glabra Pflanze mit einer Glycyrrhizinsäurekonzentration von 7 % (w/w) eingesetzt. Der wässrige Glycyrrhiza-Trockenextrakt wurde wie in Beispiel 1 beschrieben hergestellt.

Dazu wurden 120 Aufzuchtferkel in vier Versuchsgruppen zu je 10 Buchten mit jeweils 3 Ferkeln aufgeteilt. Das Futter wurde *ad libitum* verabreicht. Der Versuch startete mit 4 Wochen alten Ferkeln mit einem Gewicht von 7,7 kg.

Die positive Kontrollgruppe erhielt gewöhnliches Aufzuchtferkelfutter (Phase 1, Tag 1 - 14: Mais 32,00 %, Gerste 34,90 %, Eiweißvormischung 23 %, Sonnenblumenöl 1,00 %, Dextrose 4,00 %, Laktose 3,00 %, Ferkelprämix 2,1 %. Phase 2, Tag 15 - 56: Mais 41,00 %, Gerste 35,00 %, Soja HP 20,00 %, Sonnenblumenöl 0,50 %, Ferkelprämix 3,5 %).

Die negative Kontrollgruppe erhielt mit Polypeptid-Fungitoxinen kontaminiertes Aufzuchtferkelfutter gleicher Formulierung wie die positive Kontrollgruppe. Die Gesamtkontaminierung an Polypeptid-Fungitoxine betrug 7183,6 ppb, wobei Beauvericin zu 717 ppb, Enniatine zu 4733,6 ppb (ENN A 86 ppb, ENN A1 40 ppb, ENN B 1492 ppb, ENN B1 3111 ppb, ENN B2 4 ppb, ENN B3 0,6) und Apicidin zu 1733 ppb im finalen Aufzuchtferkelfutter vorlag. Die natürliche Kontaminierung durch Aflatoxin und Deoxynivalenol war jeweils < 1 ppb und somit vernachlässigbar.

Die zwei Testgruppen erhielten dasselbe mit Polypeptid-Fungitoxinen kontaminierte Futter wie die negative Kontrollgruppe und zusätzlich das Glycyrrhiza-Antidot in einer Einmischrate von 5 g (Testgruppe 1) und 30 g (Testgruppe 2) pro Tonne Futtermittel. Der Versuchszeitraum betrug 56 Tage.

Die Leistungsparameter Lebendgewicht und Futterverwertungsrate wurden am Ende des Versuchszeitraums bestimmt und sind in Tabelle 7 dargestellt. Die Tiere der negativen Kontrollgruppe litten dabei unter Polypeptid-Fungitoxin-Toxikose, was Fressunlust, Durchfall und insbesondere zu eine deutliche Verschlechterung der Leistungsparameter (Lebendgewicht: Verschlechterung 17,1 %; Futterverwertungsrate: Verschlechterung 6,4 %) zur Folge hatte. Die Verabreichung des Glycyrrhiza-Antidots in den beiden Testgruppen verursachte eine Reduktion der toxischen Wirkung der Polypeptid-Fungitoxine und somit eine weit weniger ausgeprägte bzw. nicht mehr vorhandene Polypeptid-Fungitoxin-Toxikose, was in beiden Einmischraten die Leistungsparameter im Vergleich zur negativen Kontrollgruppe deutlich verbesserte.

Das erfinderische Glycyrrhiza-Antidots bzw. Glycyrrhiza-Pflanzenpräparat kann somit zur Reduktion der toxischen Wirkung von wenigstens einem Polypeptid-Fungitoxin in agrarischen Produkten als auch zur Steigerung der Leistungsparameter Lebendgewicht und Futterverwertungsrate von mit Polypeptid-Fungitoxinen kontaminiertem Futter für Nutztiere, insbesondere bei Schweinen, verwendet werden sowie zur Behandlung und Prophylaxe von Polypeptid-Fungitoxin-Toxikosen.

Tabelle 7: Leistungsparameter

|  | Positive Kontrollgruppe [kg] | Negative Kontrollgruppe [kg] | Testgruppe 1 [kg] | Testgruppe 2 [kg] |
|---|---|---|---|---|
| durchschnittliches Lebendgewicht Tag 56 | 35 | 29 | 32 | 33 |
| Futterverwertungsrate [kg/kg] | 1,59 | 1,70 | 1,65 | 1,62 |

**Patentansprüche**

1.  Verwendung von wenigstens einem Glycyrrhiza-Pflanzenpräparat gewählt aus der Gruppe Mehl, wässriger Extrakt, wässriger/ethanolischer Extrakt, wässriger Trockenextrakt und wässriger/ethanolischer Trockenextrakt aus einer gesamten Glycyrrhiza-Pflanze oder aus Wurzeln der Glycyrrhiza-Pflanze, gegebenenfalls gemeinsam mit wenigstens einem Hilfsstoff, zur Reduktion der toxischen Wirkung von wenigstens einem Polypeptid-Fungitoxin in agrarischen Produkten.

2.  Verwendung nach Anspruch 1, **dadurch gekennzeichnet, dass** die Glycyrrhiza-Pflanze gewählt ist aus der Gruppe von Glycyrrhiza glabra und Glycyrrhiza uralensis.

3.  Verwendung nach Anspruch 1 oder 2, **dadurch gekennzeichnet, dass** der wässrige Trockenextrakt mit einer Glycyrrhizinsäurekonzentration von 4% (w/w) bis 10% (w/w), bevorzugterweise 7% (w/w) eingesetzt wird.

4.  Verwendung nach Anspruch 3, **dadurch gekennzeichnet, dass** wenigstens 1 g, 1 g bis 100 g, bevorzugt 7 g bis 50 g, bevorzugt 5 g bis 40 g, bevorzugt 5 g bis 30 g des wässrigen Trockenextrakts von pro Tonne agrarischem Produkt eingesetzt wird oder eine äquivalente Menge von einem der anderen Glycyrrhiza-Pflanzenpräparat nach Anspruch 1, 2 oder 3.

5.  Verwendung nach einem der Ansprüche 1 bis 4, **dadurch gekennzeichnet, dass** die toxische Wirkung von wenigstens einem Polypeptid-Fungitoxin gewählt aus der Gruppe Enniatine, insbesondere Enniatin A, Enniatin A1, Enniatin B, Enniatin B1, Enniatin B2 oder Enniatin B3; Beauvericin und Apicidin, insbesondere vollständig eliminiert wird.

6.  Verwendung nach einem der Ansprüche 1 bis 5, **dadurch gekennzeichnet, dass** das agrarische Produkt aus Nahrungs- oder Futtermitteln bestehend aus oder enthaltend wenigstens ein Erzeugnis gewählt aus der Gruppe Getreide, Mais, Reis, Soja und andere Leguminosen, Raps, Gräser, Kräuter gewählt wird.

7.  Verwendung nach einem der Ansprüche 1 bis 6, **dadurch gekennzeichnet, dass** der Hilfsstoff gewählt ist aus der Gruppe inerte Träger, Vitamine, Mineralstoffe, phytogene Substanzen, Enzyme und weitere Komponenten zur Detoxifizierung von Mykotoxinen, wie Mykotoxin-abbauende Enzyme, insbesondere Aflatoxin-Oxidasen, Ergotamin-Hydrolasen, Ergotamin-Amidasen, Zearalenon-Esterasen, Zearalenon-Lactonasen, Zearalenon-Hydrolasen, Ochratoxin-Amidasen, Fumonisin-Aminotransferasen, Fumionisin-Carboxyltransferasen, Aminopolyol-Aminoxidasen, Deoxynivalenol-Epoxidhydrolasen, Deoxynivalenol-Dehydrogenasen, Deoxynivalenol-Oxidasen, Trichothecene-Dehydrogenasen, Trichothecene-Oxidasen; und Mykotoxin-transformierende Mikroorganismen, wie z.B. DSM 11798; und Mykotoxin-bildende Stoffe, beispielsweise mikrobielle Zellwände oder anorganische Materialien, wie Bentonite.

8. Antidot zur oralen Einnahme zur Reduktion der toxischen Wirkung von wenigstens einem Polypeptid-Fungitoxin enthaltend wenigstens ein Glycyrrhiza-Pflanzenpräparat, gegebenenfalls gemeinsam mit wenigstens einem Hilfsstoff.

9. Antidot nach Anspruch 8, **dadurch gekennzeichnet, dass** das wenigstens eine Polypeptid-Fungitoxin gewählt ist aus der Gruppe Enniatine, insbesondere Enniatin A, Enniatin A1, Enniatin B, Enniatin B1, Enniatin B2 oder Enniatin B3; Beauvericin und Apicidin.

10. Antidot nach Anspruch 8 oder 9, **dadurch gekennzeichnet dass** das Glycyrrhiza-Pflanzenpräparat gewählt ist aus der Gruppe Mehl, wässriger Extrakt, wässriger/ethanolischer Extrakt, wässriger Trockenextrakt und wässriger/ethanolischer Trockenextrakt aus einer gesamten Glycyrrhiza-Pflanze oder aus Glycyrrhiza Wurzeln.

11. Antidot nach Anspruch 10, **dadurch gekennzeichnet, dass** die Glycyrrhiza-Pflanze gewählt ist aus der Gruppe von Glycyrrhiza glabra und Glycyrrhiza uralensis.

12. Antidot nach Anspruch 8 bis 11, **dadurch gekennzeichnet, dass** der wässrige Trockenextrakt 4% (w/w) bis 10% (w/w) Glycyrrhizinsäure, bevorzugterweise 7% (w/w) Glycyrrhizinsäure enthält.

13. Antidot nach einem der Ansprüche 12, **dadurch gekennzeichnet, dass** 1 mg bis 100 mg, bevorzugt 7 mg bis 50 mg, bevorzugt 5 mg bis 40 mg, bevorzugt 5 mg bis 30 mg des wässrigen Trockenextrakts pro Kilogramm Nahrungs- oder Futtermittel oder eine äquivalente Mengen von wenigstens einem der anderen Glycyrrhiza-Pflanzenpräparate nach einem der Ansprüche 10 oder 11 eingesetzt ist.

14. Antidot nach einem der Ansprüche 8 bis 13, **dadurch gekennzeichnet, dass** es mehr als 50% (w/w), bevorzugt mehr als 90% (w/w), insbesondere 100% (w/w) des wenigstens einen Glycyrrhiza-Pflanzenpräparats enthält.

15. Antidot nach einem der Ansprüche 8 bis 14, **dadurch gekennzeichnet, dass** der wenigstens eine Hilfsstoff gewählt ist aus der Gruppe inerter Träger, Vitamine, Mineralstoffe, phytogene Substanzen, Enzyme und weitere Komponenten zur Detoxifizierung von Mykotoxinen, wie Mykotoxin-abbauende Enzyme, insbesondere Aflatoxin-Oxidasen, Ergotamin-Hydrolasen, Ergotamin-Amidasen, Zearalenon-Esterasen, Zearalenon-Lactonasen, Zearalenon-Hydrolasen, Ochratoxin-Amidasen, Fumonisin-Aminotransferasen, Fumonisin-Carboxyltransferasen, Aminopolyol-Aminoxidasen, Deoxynivalenol-Epoxidhydrolasen, Deoxynivalenol-Dehydrogenasen, Deoxynivalenol-Oxidasen, Trichothecene-Dehydrogenasen, Trichothecene-Oxidasen; und Mykotoxin-transformierende Mikroorganismen, wie z.B. DSM 11798; und Mykotoxin-bildende Stoffe, beispielsweise mikrobielle Zellwände oder anorganische Materialien, wie Bentonite.

16. Verwendung eines Antidots nach einem der Ansprüche 8 bis 15 zur Herstellung eines Präparats zur Prophylaxe und/oder Behandlung von Polypeptid-Fungitoxin-Toxikosen.

17. Verwendung nach Anspruch 16, **dadurch gekennzeichnet, dass** die Polypeptid-Fungitoxin-Toxikosen verursacht werden durch Polypeptid-Fungitoxine gewählt aus der Gruppe Enniatine, insbesondere Enniatin A, Enniatin A1, Enniatin B, Enniatin B1, Enniatin B2 oder Enniatin B3; Beauvericin und Apicidin.

18. Verfahren zur akuten Behandlung eines Subjekts welches an einer Polypeptid-Fungitoxin-Toxikose leidet und/oder zur prophylaktischen Behandlung eines Subjekts zur Vorbeugung einer Polypeptid-Fungitoxin-Toxikose durch orale Verabreichung einer effektiven Menge eines Glycyrrhiza-Antidots an das Subjekt.

19. Verfahren nach Anspruch 18, **dadurch gekennzeichnet, dass** die Polypeptid-Fungitoxin-Toxikosen verursacht werden durch Polypeptid Fungitoxine gewählt aus der Gruppe Enniatine, insbesondere Enniatin A, Enniatin A1, Enniatin B, Enniatin B1, Enniatin B2 oder Enniatin B3; Beauvericin und Apicidin.

**20.** Verfahren nach einem der Ansprüche 18 oder 19, **dadurch gekennzeichnet dass** als Glycyrrhiza-Antidot ein Antidot nach wenigstens einem der Ansprüche 8 bis 15 eingesetzt wird.

**21.** Verfahren nach einem der Ansprüche 18 bis 20, **dadurch gekennzeichnet, dass** als effektive Menge des Glycyrrhiza-Antidots 1 mg bis 100 mg, bevorzugt 7 mg bis 50 mg, bevorzugt 5 mg bis 40 mg, bevorzugt 5 mg bis 30 mg eines Antidots nach Anspruch 12 pro Kilogramm Nahrungs- oder Futtermittel oder äquivalente Mengen eines Antidots nach wenigstens einem der Ansprüche 8 bis 11 eingesetzt werden.

Europäisches
Patentamt

European
Patent Office

Office européen
des brevets

# EUROPÄISCHER RECHERCHENBERICHT

Nummer der Anmeldung

EP 16 45 0032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | DATABASE WPI<br>Week 201616<br>Thomson Scientific, London, GB;<br>AN 2015-765651<br>XP002768936,<br>& CN 105 012 673 A (YIN H)<br>4. November 2015 (2015-11-04)<br>* Zusammenfassung *<br>----- | 1-21 | INV.<br>A61K36/484<br>A61P39/00<br>A61P39/02 |
| Y | DATABASE WPI<br>Week 201430<br>Thomson Scientific, London, GB;<br>AN 2014-D59547<br>XP002768937,<br>& CN 103 463 179 A (BEIJING CENT BIOLOGY CO LTD) 25. Dezember 2013 (2013-12-25)<br>* Zusammenfassung *<br>----- | 1-21 | |
| Y | DATABASE WPI<br>Week 201332<br>Thomson Scientific, London, GB;<br>AN 2013-G03486<br>XP002768938,<br>& CN 102 872 272 A (QINGDAO TIANRUI BIO-TECHNOLOGY CO LTD)<br>16. Januar 2013 (2013-01-16)<br>* Zusammenfassung *<br>-----<br><br>-/-- | 1-21 | |

RECHERCHIERTE SACHGEBIETE (IPC)

A61K
A61P

Der vorliegende Recherchenbericht wurde für alle Patentansprüche erstellt

| Recherchenort | Abschlußdatum der Recherche | Prüfer |
|---|---|---|
| München | 6. April 2017 | Thalmair, Michaela |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
..........
& : Mitglied der gleichen Patentfamilie, übereinstimmendes
Dokument

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 16 45 0032

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | CHENG G: "Healthcare licorice beverage processing method used for preventing toxin, by drying crude licorice, sterilizing, processing into hot beverage-healthcare licorice tea or extracting with water, sterilizing, cooling, and packing", WPI / THOMSON,, Bd. 2011, Nr. 16, 17. November 2010 (2010-11-17), XP002700296, * Zusammenfassung * ----- | 1-21 | |
| Y | RASHIN MOHSENI ET AL: "Antitoxin characteristic of licorice extract: the inhibitory effect on aflatoxin production in Aspergillus parasiticus", JOURNAL OF FOOD SAFETY, WILEY-BLACKWELL PUBLISHING, INC, UNITED STATES, Bd. 34, Nr. 2, 1. Januar 2014 (2014-01-01), Seiten 119-125, XP009194024, ISSN: 0149-6085 * das ganze Dokument * ----- | 1-21 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 6. April 2017 | Thalmair, Michaela |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................................
& : Mitglied der gleichen Patentfamilie, übereinstimmendes Dokument

EPO FORM 1503 03.82 (P04C03)

Seite 2 von 2

**ANHANG ZUM EUROPÄISCHEN RECHERCHENBERICHT
ÜBER DIE EUROPÄISCHE PATENTANMELDUNG NR.**

EP 16 45 0032

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-04-2017

| Im Recherchenbericht angeführtes Patentdokument | Datum der Veröffentlichung | Mitglied(er) der Patentfamilie | Datum der Veröffentlichung |
|---|---|---|---|
| CN 105012673 A | 04-11-2015 | KEINE | |
| CN 103463179 A | 25-12-2013 | KEINE | |
| CN 102872272 A | 16-01-2013 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **LIU et al.** *Zhongguo Zhong Yao Za Zhi,* 2014, vol. 39 (19), 3841ff
- **STREIT et al.** *Toxins,* 2013, vol. 5, 504 ff
- **FRISVAD et al.** *Adv. Exp. Med. Biol.,* 2006, vol. 571, 3ff
- *CHEMICAL ABSTRACTS,* 26048-05-5
- *CHEMICAL ABSTRACTS,* 11113-62-5
- *CHEMICAL ABSTRACTS,* 2503-13-1
- *CHEMICAL ABSTRACTS,* 4530-21-6
- *CHEMICAL ABSTRACTS,* 917-13-5
- *CHEMICAL ABSTRACTS,* 19914-20-6
- *CHEMICAL ABSTRACTS,* 183506-66-3
- **STREIT et al.** *Toxins,* 2012, vol. 4, 788ff
- *EFSA Journal,* 2014, vol. 12, 3802
- **MCKEE et al.** *J. Nat. Prod,* 1997, vol. 60, 431ff
- **PARK et al.** *Appl. Environ. Microbiol.,* 1999, vol. 86, 126ff
- **KARASAASLAAN ; DALGICI.** *J. Food Sci. Technol.,* 2014, vol. 51 (11), 3014ff
- **BAUER et al.** *Lehrbuch der Pharmazeutischen Technologie,* 2012, ISBN 978-3-8047-2552-2
- **GÜLDEN ; SEIBERT.** *ALTEX,* 2005, vol. 22
- **GEENS ; NIEWOLD.** *Cytotechnology,* 2011, vol. 63, 415ff